# EUROPEAN PATENT SPECIFICATION

(11) **EP 1 667 764 B1**
(45) Date of publication and mention of the grant of the patent: **26.02.2014**
(21) Application number: 04784620.9
(22) Date of filing: 16.09.2004
(51) Int. Cl.: A61N 1/05, A61N 1/04, A61B 17/34, A61B 1/04

(54) **AXIAL TO PLANAR LEAD CONVERSION DEVICE AND METHOD**
AXIAL/PLANAR-LEITUNGSKONVERSIONSVORRICHTUNG UND VERFAHREN
DISPOSITIF ET PROCEDE PERMETTANT DE CONVERTIR UN CONDUCTEUR AXIAL EN CONDUCTEUR PLAN

(30) Priority: 16.09.2003 US 503465 P
(43) Date of publication of application: 14.06.2006
(73) Proprietor: Boston Scientific Neuromodulation Corporation, Valencia, CA 91355 (US)
(72) Inventor: MEADOWS, Paul, M., Glendale, CA 91208-2240 (US); PAYNE, David, H., Llano, CA 93544 (US); BRADLEY, Kerry, Glendale, CA 91208 (US)
(74) Representative: Hermann, Gerhard
(86) International application number: PCT/US2004/030817
(87) International publication number: WO 2005/028025

(56) References cited:
- EP-A- 0 826 390
- EP-A- 1 048 270
- US-A- 5 269 810
- US-A- 5 330 523
- US-A- 5 849 033
- US-B1- 6 324 435

## Description

### Background

The present invention relates to neural stimulation and, more particularly, to leads, lead adapters and methods used with implantable stimulation systems.

Existing lead designs for spinal cord stimulation (SCS) typically consist of two types: percutaneous (or "axial") leads and laminectomy (or "planar" or "paddle") leads. A clinician introduces a percutaneous lead into the epidural space by way of a hypodermic needle, e.g., a Touhy needle or a trocar. Laminectomy leads, typically implanted by a neurosurgeon, are introduced into the epidural space after removal of a portion of the vertebrae at the region of insertion. Fewer laminectomy leads are implanted in the United States compared to percutaneous leads, due in part to the more difficult nature of the surgical placement and because neurosurgeons implant laminectomy leads, whereas implanting percutaneous leads is less surgically invasive and can be performed by an anesthesiologists.

U.S. Patent No. 3,822,708 to Zilber describes one of the first near-paddle lead designs available. US Patents 4,044,774 to Corbin et al. and 4,379,462 to Borkan et al. describe some of the first percutaneous lead designs available, including use of a stylet for placement of the lead.

U.S. Patent No. 4,285,347 to Hess describes a method for stabilizing a percutaneous epidural lead having a resilient distal end for forming a curved loop with expandable loop elements. This method can help secure the lead once the stylet is withdrawn from the lead. The method may result in improved anchoring of the lead, but may have little effect on maintaining close proximity of the lead to the dura.

U.S. Patent No. 4,519,403 to Dickhudt describes a balloon lead and inflator for unidirectionally pushing a lead against the spinal canal and spinal cord.

U.S. Patent Nos. 4,538,624 and 4,549,556 to Tarjan et al. describe a complicated lead and introduction method. The introduction method requires two needles and a surrogate lead coupled to the stimulating lead. The surrogate lead pulls the stimulating lead into position in the epidural space. Molded projections on the stimulating lead act to anchor the lead in the epidural space.

U.S. Patent No. 5,417,719 to Hull et al. describes a standard paddle lead with various configurations of rectangular electrodes. The paddle is flat and has no features that would cause it to be held closely to the dura. This is the conventional style of paddle lead, which suffers from the problems related to separation from the dural surface.

U.S. Patent Nos. 5,501,703 and 5,643,330 to Holsheimer et al. also describe a paddle lead with multiple electrodes. No method is suggested to hold the paddle against the dura.

U.S. Patent No. 5,733,322 to Starkebaum describes a percutaneous lead with an extension that extends distally beyond the most distal electrode. When implanted, the extension is positioned between the dura and spinal canal wall where they are in contact, thus holding the extension in place. Dimples on the extension can aid in anchoring the lead in place. As with standard percutaneous leads, the '322 lead has a diameter allowing it to fit an introduction needle or trocar.

U.S. Patent 6,163,727 to Errico describes a hook-shaped spinal cord lead assembly that is secured about a spinous process. This lead is subject to poor control of electrode position with respect to the dura, as anatomical variation would be almost impossible to accommodate, resulting in unpredictable location of the electrode array over the dura. In addition, this lead would not be easily positioned axially with respect to the optimal stimulation site.

U.S. Patent No. 6,175,769 to Errico et al. describes a lead assembly having laterally extending parts, which are intended to aid in preventing displacement of the lead. However, the lateral parts have, in one form, suture holes so that the lead can be secured to the spinous process. While this might hold the lead in a fixed position with respect to the vertebrae, it may also have the effect of pulling the lead away from the dura, resulting in higher stimulation currents being required. Also, like the design of the '727 patent (also by Errico), the lead would not be optimally located axially with respect to the desired location of the nerves to be stimulated.

U.S. Patent No. 6,308,103 to Gielen describes a self-centering paddle lead and method. This paddle lead includes a pivotal member as a feature on the back of the lead. The pivotal member is intended to hold the lead close to the spinal cord and to prevent lead migration. The pivotal member is meant to be inflatable with a hardening agent, such as silicone rubber. It can be seen that this feature must be designed into each lead that would take advantage of this feature or be secured to the back of a paddle lead with sutures, adhesives or other attachment means.

U.S. Patent No. 6,309,401 to Redko et al. describes a specialized, flattened needle for introduction of a paddle style lead. This allows the lead to be inserted by an anesthesiologist, whereas conventional paddle leads are typically only implanted by neurosurgeons because the surgery needed to expose the dura of the spinal cord is extensive and includes removing vertebrae sections. US Patent No. 6,249,707 to Kohnen et al. describes a needle for introducing a paddle-type lead and a paddle lead adapted to accept a stiffening member. This is a variation of the '401 patent by Redko, with the addition of the stiffening member used during the positioning of the lead. Document EP 0 826 390 discloses a coupling element for a distal end of a surgical electrode.

Despite the popularity of percutaneous leads, problems exist that are not well addressed by current designs on the market. Percutaneous leads may be constructed to an optimal lead length, width and electrode spacing, but all of the electrodes are placed in a linear, axial array. Sometimes it is desirable to have two, linear, axial electrode arrays that are placed in parallel. To achieve such parallel placement, two percutaneous leads are implanted side-by-side. Achieving exact, parallel placement of the two electrode arrays, however, can often be a difficult procedure. A further difficulty with leads in general, and especially with percutaneous leads, is that over time, a percutaneous lead may migrate, changing the position of the electrode array relative to the target tissue to be stimulated, resulting in variations in the level of paresthesia to the patient.

### Brief Summary of the Invention

The present invention addresses a need to transform a percutaneous or axial lead into a paddle-type or planar lead. This is accomplished with an adapter having at least two channels for accepting into the channels at least two percutaneous (axial) leads. The channels are preferably open channels that have longitudinal openings along the surface of the adapter, which longitudinal openings may be used to snap or insert the axial lead into the channel. The channels are appropriately dimensioned to accept the axial leads.

In the present invention, the adapter includes two or more lead accepting channels.

The channels may be essentially circular in cross-section, having an identifiable diameter. Of course, the channels may have other cross-sectional shapes to accommodate the particular exterior shape of axial leads. The channels are placed in parallel. This advantageously sets the spacing precisely between electrode arrays because the pre-aligned and formed channels on the adapter force an exact parallel placement of the two or more electrode arrays within the adapter. In addition, the separate, percutaneous leads placed into the parallel channels of an adapter may be offset. That is, the distal lead tip of one axial lead may be placed flush against the end of one channel, whereas a second axial lead may not be flush against the end of a second channel. If the first and second axial leads have identically spaced electrodes in an electrode array, then the corresponding two electrodes between electrode arrays will be offset from each other after insertion into the adapter. This electrode placement flexibility is not available with a conventional paddle that necessarily fixes all electrodes on the paddle into a predetermined position during manufacturing of the paddle.

The paddle may be fitted with a placement (or spacing) device that helps to position the paddle with respect to the target tissue to be stimulated. The placement device may be a separate piece that may be optionally attached to the paddle before implantation or, alternatively, the placement device may be permanently integral to the adapter. The placement device may be attached to the adapter by a number of attachments means.

The adapter may be solid, semi-solid or fillable. Likewise, the placement device may be solid, semi-solid or fillable. If fillable, the adapter or placement device may be inflated with a biocompatible gas, e.g., air, or a biocompatible liquid such saline solution or oil.

In one aspect, planar leads are provided comprising: axial leads capable of being used as a stand alone lead; an adapter having at least two channels dimensioned to accept and capture axial leads, wherein the axial leads are inserted into the adapter channels to provide planar leads.

In another aspect, a method is provided for adapting a percutaneous (axial) lead into a planar lead. The method comprises: providing free-standing axial leads; providing an adapter having at least two channels, wherein the channels are dimensioned to accept and capture the axial leads; and inserting the axial leads into the adapter channels to form a planar lead.

### Brief Description of the Drawings

The above and other aspects of the present invention will be more apparent from the following more particular description thereof, presented in conjunction with the following drawings wherein:
FIG. 1A shows a perspective view of one embodiment of an adapter, in accordance with the present invention;
FIG. 1B shows another embodiment of an adapter, in accordance with the present invention, with two percutaneous (axial) leads inserted into the adapter;
FIG. 1C depicts an alternative example of an adapter and matching percutaneous (axial) lead, which is not in accordance with the present invention;
FIG. 1D depicts the adapter and lead shown in FIG. 1C with the lead inserted in the channel of the adapter to yield a planar or paddle-type lead;
FIG. 1E depicts another embodiment of an adapter with three channels, in accordance with the present invention;
FIG. 2A shows a top view of an adapter with the channel running the entire length of the adapter, which is not part of the invention,
FIG. 2B shows a front view of the device of FIG. 1 A;
FIG. 3 shows a bottom view of another embodiment of an adapter, showing three placement devices attached to the adapter, in accordance with the present invention;
FIG. 4A shows a top view of yet another embodiment of an adapter, in accordance with the present invention, with means for securing a placement (positioning) device(s);
FIG. 4B shows a cross-sectional view taken along line 4B-4B of the adapter shown in FIG. 4A;
FIGS. 5A and 5B show cross-sectional views depicting embodiments of placement devices that can attach to adapters; and
FIGS. 6A and 6B show cross-sectional views of additional embodiments of placement devices.

Corresponding reference characters indicate corresponding components throughout the several views of the drawings.

### Detailed Description of Preferred Aspects of the Invention

The following description is of the best mode presently contemplated for carrying out the invention. This description is not to be taken in a limiting sense, but is made merely for the purpose of describing the general principles of the invention. The scope of the invention should be determined with reference to the claims.

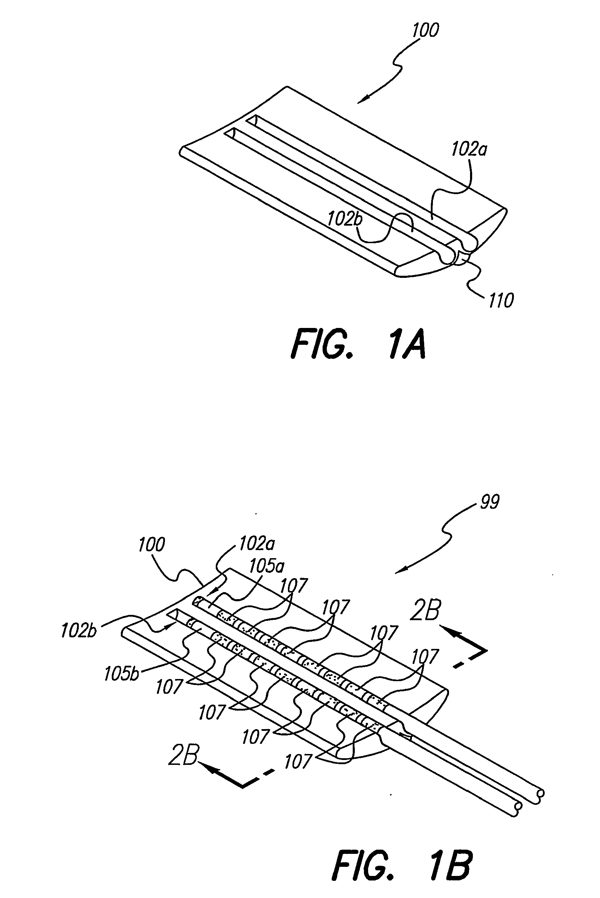

FIGS. 1A and 1B show one embodiment of an axial to planar lead adapter 100, in accordance with the present invention. The axial to planar lead adapters of the present invention, hereinafter referred to simply as adapters, have at least two open channels, e.g., 102a or 102b, which can receive a portion of a percutaneous (axial) lead 105a and/or 105b. The percutaneous leads 105a and 106b each have spaced-apart electrodes 107 forming electrode arrays on each lead. FIG. 1B shows an embodiment of an adapter with leads 105a and 105b positioned in the adapter within channels 102a and 102b, respectively, of adapter 100. The leads 105a and 105b are inserted into the adapter by pressing or snapping them into the open channels 102a and 102b, respectively, in adapter 100, thereby converting the percutaneous leads 105a and 105b, with the adapter 100, into a planar or paddle-type lead 99. Because of the shape of the channels of the adapter embodiment shown in FIGS. 1A and 1B, i.e., with a surface opening width that is slightly less than the largest width of the channel, as measured inside the adapter, the leads can be captured securely after being snapped into the channels. Other adapter embodiments may have a wider surface channel opening width then as depicted in FIGS. 1A and 1B, as these embodiments are not intended to be limiting.

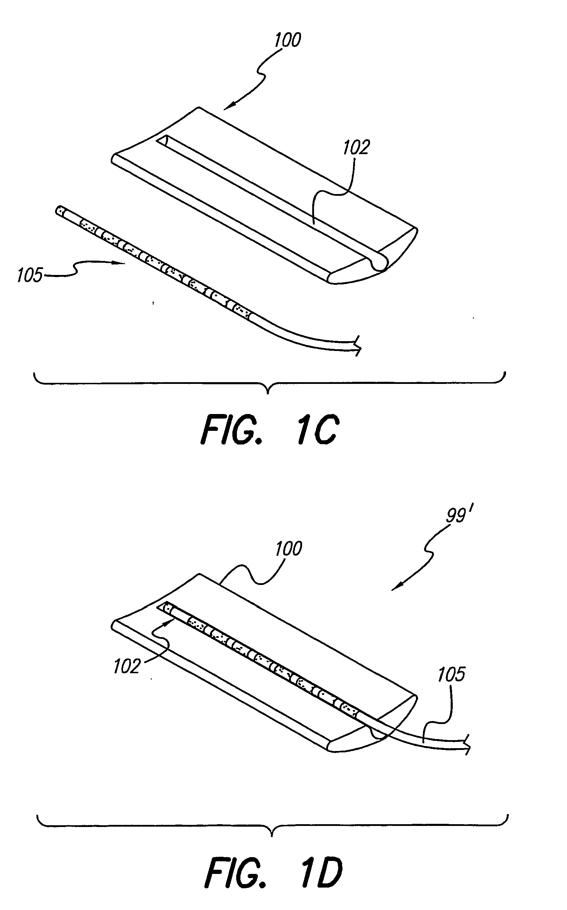

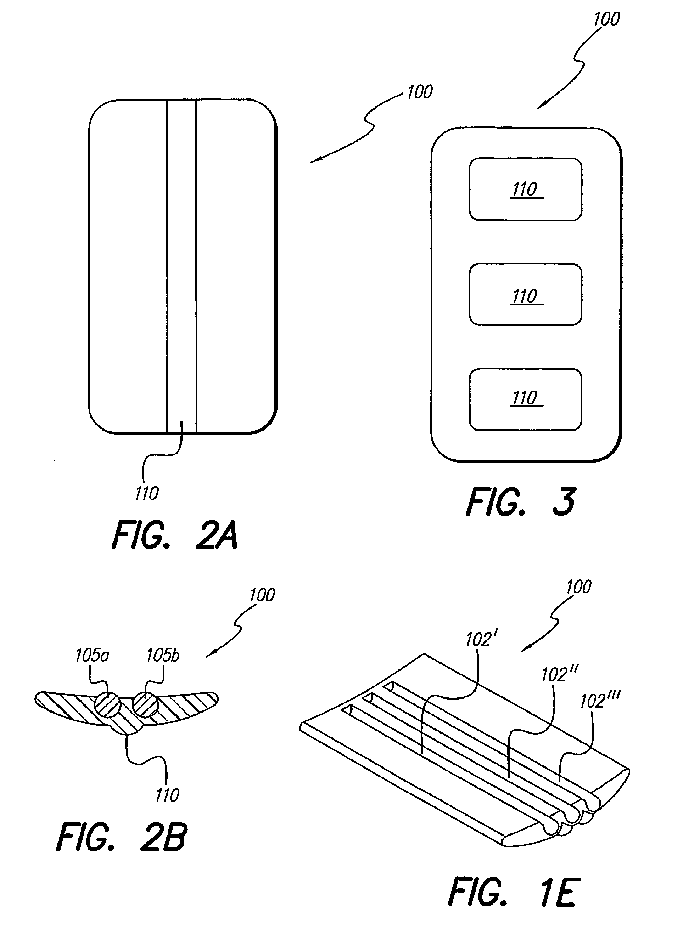

Referring to FIG. 1C, which is not part of the invention, it is noted that an adapter 100 may include just one channel 102, to transform a single, stand-alone, axial lead 105 into a planar lead or, alternatively, adapter 100 may include two, three (see FIGS. 1B and 1E) or more channels to transform two, three or more, stand-alone, axial leads into a single planar lead. FIG. 1D shows the axial lead 105 placed into channel 102 to transform the free-standing axial lead 105 into a planar lead 99'. Thus, stand-alone axial lead 105 may be placed in channel 102 (FIG. 1 C). Stand alone, axial leads 105a or 105b may be placed into channels 102a or 102b (FIG. 1 B). Three stand-alone axial leads may be placed into channels 102', 102" and 102"', which channels are as shown in FIG. 1E.

An axial lead may be inserted into a channel to use the entire channel or, alternatively, the lead may be advantageously positioned to use only a part of the channel to create an electrode array (or electrode) offset between multiple leads as shown in FIG. 1B. As seen in the example of FIG. 1B, lead 105a is inserted so it is flush with the end of channel 102a, filling it completely, and lead 105b is shown inserted into channel 102b so that an electrode 107 of lead 105b is offset from a corresponding electrode 107 on lead 105a. In the adapter embodiment shown in FIG. 1B, the length of adapter 100 is just long enough to hold the active portion of the lead, i.e., the portion of the lead having the electrodes, but the adapter 100 may be made longer or shorter, as desired. Variations in current distribution can be attained by altering the placement and positions of leads 105a and 105b within the channels 102a and 102b.

The channels 102, 102a, 102b, 102', 102", and 102'" can be made with different diameters or cross-sectional shapes to accommodate percutaneous leads with various size diameters and/or shapes. For instance, channels 102a and 102b may have different diameters or cross-sectional shapes. To facilitate matching of appropriate leads of various diameters or cross-sectional shapes with the properly dimensioned channels, the adapter channels and individual axial leads may be marked with embedded labels, lettering or color coding to identify such leads and/or channels. In addition, adapters with more than one channel can be manufactured to have various separation distances between channels to accommodate variations in spinal cord dimensions and desired lead positions with respect to dorsal midline on the dural surface.

In some embodiments, the axial to planar lead adapter 100 may have a rib or a placement device 110 to aid in the placement of the adapter. The placement device 110 is used to force the leads, e.g., 105, 105a, 105b, to be located close to the dorsal surface of the dura so that the distance between the electrodes 107 and the target nerve fibers is reduced to a minimum.

A placement device 110 or devices may be an integral part of the adapter 100, as shown in FIGS. 1A, 2B and 3. FIG. 2A shows a top view of an adapter aspect having a channel 110 running the entire length of an adapter. FIG. 2B shows a cross-sectional view of the adapter in FIG. 1B along line 2B-2B. If the adapter 100 is molded, the placement device 110 and adapter 100 may be molded as one piece so that the placement device is permanently integrated. The placement device 110 may be shaped to be continuous along the length of adapter 100 or, alternatively, the placement device 110 may be composed of shaped portions that protrude out from a surface of the adapter 100, as indicated in FIG. 3. Employing placement device 110 or devices can be useful to accommodate various sized and shaped spaces between the dura and the spinal canal wall.

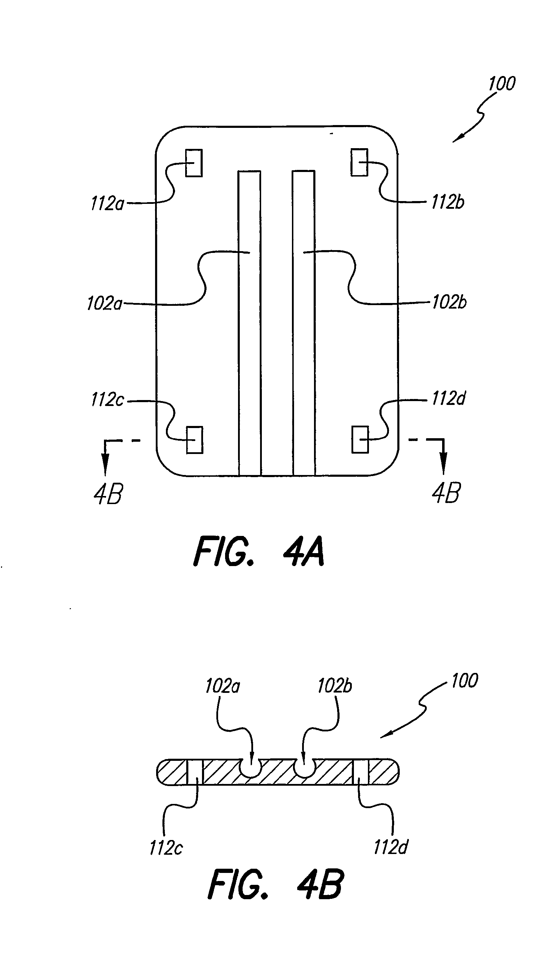

In another embodiment, when not integrally formed with the adapter, the placement device(s) 110 may be one or multiple individual pieces that can be attached and secured to adapter 100 by various attachment means. For instance, as shown in FIGS. 4A and 4B, adapter 100 may have features such as slots or holes 112a, 112b, 112c and 112d to allow a placement device or devices to be attached. FIG. 4A is a top view of an embodiment of the adapter having two channels 102a and 102b. FIG. 4B shows a cross-sectional view of the adapter of FIG. 4A along line 4B-4B.

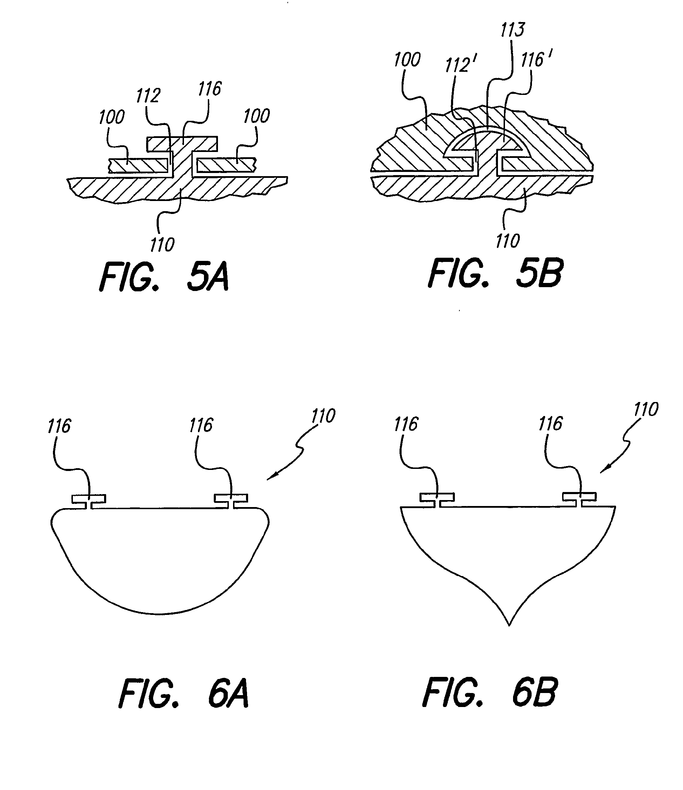

As shown in FIGS. 5A, the placement device 110 may have a locking protrusion 116 that fits through a hole 112 and therefore locks the placement device 110 to the adapter 100. In another embodiment, as shown in FIG. 5B, the placement device 110 may have a protrusion 116' that is inserted through the hole 112' and fits inside cavity 113, which cavity is within the adapter 100. The locking protrusion 116 or 116' may be shaped as a flat-head surface (FIG. 5A) or a curved surface (FIG. 5B) similar to the profile of a pan-head screw. The hole 112 (FIG. 5A) in the adapter 100 may have a counter-bore such that the protrusion 116 is set flush with the adapter surface to assume a smooth profile. Protrusion 116 or 116' may be dimensioned to be easily pulled through or pressed through the hole 112 or 112' of adapter 100 either with the hand or with an implement such as a pair of forceps. The protrusion, of course, is preferably made from a deformable insulating material such as a silicone or polyurethane that may be molded. The dimensional difference between the hole size and the protrusion size are depicted as pronounced in FIGS. 5A and 5B for illustrative purposes but the actual dimensional difference may be much smaller. Other means for securing placement device 110 to adapter 100 include, without limitation, adhesive attachment, surgical staples and sutures, among other forms of securing means.

The placement device 110 may be made in a variety of external shapes. For example, as shown in FIG. 6A, placement device 110 can be roughly hemispherical in cross-section or, as shown in FIG. 6B, it can be roughly conical. Protrusions 116 may be employed to attach the placement device 110 to the adapter 100. Many other shapes of placement device 110 are also suitable for the purpose. The placement device 110 may comprise multiple, separate sections attached to the adapter 100, as shown in FIG. 3. Or, in one embodiment, the placement device 110 may be a single section running the length of the adapter, as shown in FIG. 2B.

The placement device 110 may be solid, semi-solid, or may have an inflatable or fillable internal space, e.g., a bladder. The bladder may be inflated by injecting biocompatible liquids or gases, such as saline solution, oil, or air into the bladder. It is preferred that any liquid chosen remains in its liquid state over a long period of time so that the placement device remains compliant. The adapter 100 may be similarly a solid, semi-solid, or fillable as described for the placement device 110.

The adapter 100 may be made of flexible biocompatible lead material such as implantable grade silicone rubber. Silicone rubber is a common biocompatible lead material and can withstand repeated steam and gas sterilization. Use of other materials or combinations of materials are also possible. For example, implantable grade polyurethanes commonly used to fabricate leads may also be used to make the adapter 100.

Adapters 100 and placement devices 110 of the present invention may be manufactured by a variety of methods, including but not limited to various injection and overmolding techniques.

In sum, an adapter is provided for transforming free-standing axial leads to planar leads. In addition, planar leads are provided that combines at least two free-standing axial leads with an adapter having at least two leads accepting channels to convert axial leads into functional planar leads. When the adapter has two or more channels, the electrode arrays of the two or more leads may be variably staggered on the adapter to provide an electrode offset. In another aspect of the invention, a method is provided for transforming axial leads into planar leads the method comprising: providing free-standing axial leads; providing an adapter having at least two channels wherein the channels are dimensioned to accept and capture axial leads; and inserting axial leads into the adapter channels.

In use, medical facilities such as hospitals may use an available stand-alone percutaneous leads and flexibly adapt them to various paddle lead and electrode configurations. Advantageously, the adapters 100 can be used to transform available matching percutaneous leads immediately into planar or paddle leads. The adapters can be made into sets where the adapters are provided with channels having different sizes to accommodate different sized leads. The leads may be advantageously positioned within the adapter channels to provide an electrode stagger or offset between different leads. In addition, a set of adapters having two or more channels may be easily manufactured to be available with various separation distances between channels, again providing further electrode position selectivity. Advantageously, the adapters may also be made available in different sizes and aspects by employing placement devices of different shapes and sizes fitted to the adapters in order to further accommodate individual anatomic variation.

While the invention herein disclosed has been described by means of specific embodiments and applications thereof, numerous modifications and variations could be made thereto by those skilled in the art without departing from the scope of the invention set forth in the claims.

## Claims

1. A lead adapter (100) configured to transform at least two axial leads (105, 105a, 105b) to a planar lead (99, 99'), the adapter comprising at least two open channels (102, 102a, 102b) placed in parallel and extending longitudinally along a surface of the adapter, the at least two open channels each having a longitudinal opening that extends along the surface of the adapter, the at least two open channels each configured for receiving a distal portion of a different one of the at least two axial leads, the received portions of the at least two axial leads having a linear axial array of spaced-apart electrodes, wherein the at least two open channels are configured for receiving the at least two axial leads such that the linear, axial arrays of spaced-apart electrodes of the at least two axial leads are placed in parallel along the surface of the adapter with the spaced-apart electrodes of each of the at least two axial leads exposed through the longitudinal opening in the open channel.

2. The lead adapter (100) of claim 1, wherein the at least two open channels (102, 102a, 102b) are shaped with surface opening widths that are slightly less than the largest widths of the open channels, as measured inside the lead adapter.

3. The lead adapter (100) of claim 2 wherein at least one of the at least two open channels (102, 102a, 102b) extends completely across the adapter (100) from one edge to another edge.

4. The lead adapter (100) of claim 2 wherein at least one of the at least two open channels (102, 102a, 102b) has a cross-section with an identifiable diameter.

5. The lead adapter (100) of claim 1 wherein the adapter (100) is at least one of solid, semi-solid and fillable.

6. The lead adapter (100) of claim 5 wherein the adapter (100) is filled with at least one of a biocompatible liquid and gas.

7. The lead adapter (100) of claim 6 wherein the adapter (100) is filled with at least one of saline solution, oil and air.

8. The lead adapter (100) of claim 1 further including a placement device (110).

9. The lead adapter (100) of claim 8 wherein the placement device (110) is integral to the lead adapter (100).

10. The lead adapter (100) of claim 8 wherein the placement device (110) is optionally attachable to the lead adapter (100).

11. The lead adapter (100) of claim 8 wherein the placement device (100) includes means for securing (116,116') the placement device (110) to the lead adapter (100).

12. The lead adapter (100) of claim 8 wherein the placement device (110) is at least one of solid, semi-solid and fillable.

13. The lead adapter (100) of claim 12 wherein the placement device (110) is filled with at least one of a biocompatible liquid and gas.

14. The lead adapter (100) of claim 12 wherein the placement device (110) is filled with at least one of saline solution, oil and air.

15. A method of adapting free-standing axial leads (105, 105a, 105b) to a planar lead (99,99'), the method comprising:
providing a first free-standing, axial lead (105, 105a, 105b) having a linear, axial array of spaced-apart electrodes;
providing a second free-standing, axial lead (105, 105a, 105b) having a linear, axial array of spaced-apart electrodes;
providing an adapter (100) comprising at least two open channels (102, 102a, 102b) placed in parallel and extending longitudinally along a surface of the adapter, wherein the at least two open channels each have a longitudinal opening that extends along the surface of the adapter; and
inserting the first axial lead into one of the at least two open channels and inserting the second axial lead into another of the at least two open channels such that the spaced-apart electrodes of the first axial lead and the spaced-apart electrodes of the second axial lead are placed in parallel along the surface of the adapter with the spaced-apart electrodes of each of the at least two axial leads exposed through the longitudinal opening in the open channel.

16. A planar lead (99, 99') comprising:
a first axial lead (105, 105a) configured to be used as a stand-alone lead;
a second axial lead (105, 105a) configured to be used as a stand-alone lead ;and the adapter (100) of claim 1;
wherein the first axial lead is inserted into one of the at least two open channels (102, 102a, 102', 102'") of the adapter and wherein the second axial lead is inserted into another of the at least two open channels (102, 102a, 102', 102"') of the adapter to provide a planar lead (99,99').

17. The lead adapter (100) of claim 1, wherein the at least two open channels (102, 102a, 102b) are configured for receiving the at least two axial leads (105, 105a, 105b) such that the linear, axial arrays of spaced-apart electrodes of the at least two axial leads (105, 105a, 105b) are placed in parallel along the surface of the adapter and are longitudinally offset from one another.

18. The lead adapter (100) of claim 1, wherein the adapter has a length that is just long enough to hold the portion of at least one of the at least two axial leads (105, 105a, 105b) having the linear, axial array of spaced-apart electrodes.

## Patentansprüche

1. Leitungsadapter (100) zum Umwandeln mindestens zweier axialer Leitungen (105,105a,105b) in eine planare Leitung (99,99'), wobei der Adapter mindestens zwei offene Kanäle (102,102a,102b) aufweist, die parallel angeordnet sind und sich der Länge nach entlang einer Oberfläche des Adapters erstrecken, wobei jeder der mindestens zwei offenen Kanäle eine Längsöffnung aufweist, die sich entlang der Oberfläche des Adapters erstreckt, wobei jeder der mindestens zwei offenen Kanäle konfiguriert ist, einen distalen Abschnitt jeweils einer unterschiedlichen der mindestens zwei axialen Leitungen aufzunehmen, wobei die aufgenommenen Abschnitte der mindestens zwei axialen Leitungen eine lineare axiale Anordnung beabstandeter Elektroden aufweisen, wobei die mindestens zwei offenen Kanäle konfiguriert sind, die mindestens zwei axialen Leitungen aufzunehmen, derart, dass die linearen axialen Anordnungen beabstandeter Elektroden der mindestens zwei axialen Leitungen parallel entlang der Oberfläche des Adapters angeordnet sind, wobei die beabstandeten Elektroden jeder der mindestens zwei axialen Leitungen durch die Längsöffnung in dem offenen Kanal freiliegen.

2. Leitungsadapter (100) nach Anspruch 1, wobei die mindestens zwei offenen Kanäle (102,102a,102b) so gestaltet sind, dass die Breiten der Öffnungen an der Oberfläche etwas geringer sind als die größten Breiten der offenen Kanäle im Innern des Leitungsadapters.

3. Leitungsadapter (100) nach Anspruch 2, wobei mindestens einer der mindestens zwei offenen Kanäle (102,102a,102b) sich über den gesamten Adapter (100) von einer Kante zur anderen Kante erstreckt.

4. Leitungsadapter (100) nach Anspruch 2, wobei mindestens einer der mindestens zwei offenen Kanäle (102,102a,102b) einen Querschnitt mit einem identifizierbaren Durchmesser aufweist.

5. Leitungsadapter (100) nach Anspruch 1, wobei der Adapter (100) massiv und/oder halbmassiv und/oder füllbar ist.

6. Leitungsadapter (100) nach Anspruch 5, wobei der Adapter (100) mit einer biokompatiblen Flüssigkeit und/oder einem biokompatiblen Gas gefüllt ist.

7. Leitungsadapter (100) nach Anspruch 6, wobei der Adapter (100) mit Salzlösung und/oder Öl und/oder Luft gefüllt ist.

8. Leitungsadapter (100) nach Anspruch 1, ferner mit einer Positionierungsvorrichtung (110).

9. Leitungsadapter (100) nach Anspruch 8, wobei die Positionierungsvorrichtung (110) integral mit dem Leitungsadapter (100) ist.

10. Leitungsadapter (100) nach Anspruch 8, wobei die Positionierungsvorrichtung (110) optional an dem Leitungsadapter (100) anbringbar ist.

11. Leitungsadapter (100) nach Anspruch 8, wobei die Positionierungsvorrichtung (110) Mittel (116,116') zum Befestigen der Positionierungsvorrichtung (110) an dem Leitungsadapter (100) aufweist.

12. Leitungsadapter (100) nach Anspruch 8, wobei die Positionierungsvorrichtung (110) massiv und/oder halbmassiv und/oder füllbar ist.

13. Leitungsadapter (100) nach Anspruch 12, wobei die Positionierungsvorrichtung (110) mit einer biokompatiblen Flüssigkeit und/oder einem biokompatiblen Gas gefüllt ist.

14. Leitungsadapter (100) nach Anspruch 12, wobei die Positionierungsvorrichtung (110) mit Salzlösung und/oder Öl und/oder Luft gefüllt ist.

15. Verfahren zum Anpassen freier axialer Leitungen (105,105a,105b) an eine planare Leitung (99,99') mit den folgenden Schritten:
Bereitstellen einer ersten freien axialen Leitung (105,105a,105b), die eine lineare axiale Anordnung beabstandeter Elektroden aufweist;
Bereitstellen einer zweiten freien axialen Leitung (105,105a,105b), die eine lineare axiale Anordnung beabstandeter Elektroden aufweist;
Bereitstellen eines Adapters (100), der mindestens zwei offene Kanäle (102,102a,102b) aufweist, die parallel angeordnet sind und sich der Länge nach entlang einer Oberfläche des Adapters erstrecken, wobei jeder der mindestens zwei offenen Kanäle eine Längsöffnung aufweist, die sich entlang der Oberfläche des Adapters erstreckt; und
Einführen der ersten axialen Leitung in einen der mindestens zwei offenen Kanäle und Einführen der zweiten axialen Leitung in den anderen der mindestens zwei offenen Kanäle, derart, dass die beabstandeten Elektroden der ersten axialen Leitung und die beabstandeten Elektroden der zweiten axialen Leitung parallel entlang der Oberfläche des Adapters angeordnet sind, wobei die beabstandeten Elektroden jeder der mindestens zwei axialen Leitungen durch die Längsöffnung in dem offenen Kanal freiliegen.

16. Planare Leitung (99,99') mit:
einer ersten axialen Leitung (105,105a), die konfiguriert ist, als eine Einzelleitung verwendet zu werden;
einer zweiten axialen Leitung (105,105a), die konfiguriert ist, als eine Einzelleitung verwendet zu werden; und
dem Adapter (100) nach Anspruch 1;
wobei die erste axiale Leitung in einen der mindestens zwei offenen Kanäle (102,102a,102',102''') des Adapters eingeführt ist und wobei die zweite axiale Leitung in den anderen der mindestens zwei offenen Kanäle (1 02,102a, 102',102''') des Adapters eingeführt ist, um eine planare Leitung (99,99') bereitzustellen.

17. Leitungsadapter (100) nach Anspruch 1, wobei die mindestens zwei offenen Kanäle (102,102a,102b) konfiguriert sind, die mindestens zwei axialen Leitungen (105,105a,105b) aufzunehmen, derart, dass die linearen axialen Anordnungen beabstandeter Elektroden der mindestens zwei axialen Leitungen (105,105a,105b) parallel entlang der Oberfläche des Adapters angeordnet sind und in Längsrichtung zueinander versetzt angeordnet sind.

18. Leitungsadapter (100) nach Anspruch 1, wobei der Adapter eine Länge hat, die gerade lang genug ist, um den Abschnitt mindestens einer der mindestens zwei axialen Leitungen (105,105a,105b), der die lineare axiale Anordnung beabstandeter Elektroden aufweist, zu halten.

## Revendications

1. Adaptateur de broches (100) configuré pour transformer au moins deux broches axiales (105, 105a, 105b) en une broche plate (99, 99'), l'adaptateur comprenant au moins deux canaux ouverts (102, 102a, 102b) placés en parallèle et s'étendant longitudinalement le long d'une surface de l'adaptateur, les au moins deux canaux ouverts ayant chacun une ouverture longitudinale qui s'étend le long de la surface de l'adaptateur, les au moins deux canaux ouverts étant configurés pour recevoir une partie distale d'une différente des au moins deux broches axiales, les parties reçues des au moins deux broches axiales ayant un réseau axial linéaire d'électrodes espacées, dans lequel les au moins deux canaux ouverts sont configurés pour recevoir les au moins deux broches axiales de telle manière que les réseaux axiaux linéaires d'électrodes espacées des au moins deux broches axiales sont placés en parallèle le long de la surface de l'adaptateur avec les électrodes espacées de chacune des au moins deux broches axiales exposées par l'ouverture longitudinale dans le canal ouvert.

2. Adaptateur de broches (100) selon la revendication 1, dans lequel les au moins deux canaux ouverts (102, 102a, 102b) sont formés avec des largeurs d'ouverture de surface qui sont légèrement inférieures aux largeurs les plus grandes des canaux ouverts, telles que mesurées à l'intérieur de l'adaptateur de broches.

3. Adaptateur de broches (100) selon la revendication 2 dans lequel au moins l'un des au moins deux canaux ouverts (102, 102a, 102b) s'étend complètement d'un bord à l'autre de l'adaptateur (100).

4. Adaptateur de broches (100) selon la revendication 2 dans lequel au moins l'un des au moins deux canaux ouverts (102, 102a, 102b) a une section transversale avec un diamètre identifiable.

5. Adaptateur de broches (100) selon la revendication 1 dans lequel l'adaptateur (100) est au moins l'un de solide, semi-solide et remplissable.

6. Adaptateur de broches (100) selon la revendication 5 dans lequel l'adaptateur (100) est rempli avec au moins l'un d'un liquide et d'un gaz biocompatibles.

7. Adaptateur de broches (100) selon la revendication 6 dans lequel l'adaptateur (100) est rempli avec au moins l'un parmi une solution saline, de l'huile et de l'air.

8. Adaptateur de broches (100) selon la revendication 1 comprenant en outre un dispositif de placement (110).

9. Adaptateur de broches (100) selon la revendication 8 dans lequel le dispositif de placement (110) est d'un seul bloc avec l'adaptateur de broche (100).

10. Adaptateur de broches (100) selon la revendication 8 dans lequel le dispositif de placement (110) peut en option être fixé sur l'adaptateur de broche (100).

11. Adaptateur de broches (100) selon la revendication 8 dans lequel le dispositif de placement (110) inclut des moyens pour fixer (116, 116') le dispositif de placement (110) sur l'adaptateur de broche (100).

12. Adaptateur de broches (100) selon la revendication 8 dans lequel le dispositif de placement (110) est au moins l'un de solide, semi-solide et remplissable.

13. Adaptateur de broches (100) selon la revendication 12 dans lequel le dispositif de placement (110) est rempli avec au moins l'un d'un liquide et d'un gaz biocompatibles.

14. Adaptateur de broches (100) selon la revendication 12 dans lequel le dispositif de placement (110) est rempli avec au moins l'un parmi une solution saline, de l'huile et de l'air.

15. Procédé d'adaptation de broches axiales autonomes (105, 105a, 105b) sur une broche plate (99, 99'), le procédé comprenant :
de fournir une première broche axiale autonome (105, 105a, 105b) ayant un réseau axial linéaire d'électrodes espacées ;
de fournir une seconde broche axiale autonome (105, 105a, 105b) ayant un réseau axial linéaire d'électrodes espacées ;
de fournir un adaptateur (100) comprenant au moins deux canaux ouverts (102, 102a, 102b) placés en parallèle et s'étendant longitudinalement le long d'une surface de l'adaptateur, dans lequel les au moins deux canaux ouverts ont chacun une ouverture longitudinale qui s'étend le long de la surface de l'adaptateur ; et
d'insérer la première broche axiale dans l'un des au moins deux canaux ouverts et d'insérer la seconde broche axiale dans l'autre des au moins deux canaux ouverts de telle manière que les électrodes espacées de la première broche axiale et les électrodes espacées de la seconde broche axiale sont placées parallèlement le long de la surface de l'adaptateur avec les électrodes espacées de chacune des au moins deux broches axiales exposées par l'ouverture longitudinale dans le canal ouvert.

16. Broche plate (99, 99') comprenant :
une première broche axiale (105, 105a) configurée pour être utilisée comme une broche seule ;
une seconde broche axiale (105, 105a) configurée pour être utilisée comme une broche seule ; et
l'adaptateur (100) selon la revendication 1 ;
dans lequel la première broche axiale est insérée dans l'un des au moins deux canaux ouverts (102, 102a, 102', 102"') de l'adaptateur et dans lequel la seconde broche axiale est insérée dans l'autre des au moins deux canaux ouverts (102, 102a, 102', 102"') de l'adaptateur pour former une broche plate (99, 99').

17. Adaptateur de broches (100) selon la revendication 1, dans lequel les au moins deux canaux ouverts (102, 102a, 102b) sont configurés pour recevoir les au moins deux broches axiales (105, 105a, 105b) de telle manière que les réseaux axiaux linéaires d'électrodes espacées des au moins deux broches axiales (105, 105a, 105b) sont placés parallèlement le long de la surface de l'adaptateur et sont décalés longitudinalement l'un de l'autre.

18. Adaptateur de broches (100) selon la revendication 1, dans lequel l'adaptateur a une longueur qui est juste suffisamment longue pour contenir la partie d'au moins une des deux broches axiales (105, 105a, 105b) ayant le réseau axial linéaire d'électrodes espacées.
